(19) Europäisches Patentamt
European Patent Office
Office européen des brevets

(11) Veröffentlichungsnummer: **0 590 416 A1**

# (12) EUROPÄISCHE PATENTANMELDUNG

(21) Anmeldenummer: **93114858.9**

(22) Anmeldetag: **15.09.93**

(51) Int. Cl.[5]: **C07D 409/12**, A01N 47/36, C07D 333/38

(30) Priorität: **28.09.92 DE 4232417**

(43) Veröffentlichungstag der Anmeldung: **06.04.94 Patentblatt 94/14**

(84) Benannte Vertragsstaaten: **BE CH DE FR GB IT LI NL**

(71) Anmelder: **BAYER AG**

**D-51368 Leverkusen(DE)**

(72) Erfinder: **Gesing, Ernst R.F., Dr.**
**Trillser Graben 4**
**D-40699 Erkrath(DE)**
Erfinder: **Santel, Hans-Joachim, Dr.**
**Grünstrasse 9a**
**D-51371 Leverkusen(DE)**
Erfinder: **Lürssen, Klaus, Dr.**
**August-Kierspel-Strasse 145**
**D-51469 Bergisch Gladbach(DE)**
Erfinder: **Schmidt, Robert R., Dr.**
**Im Waldwinkel 110**
**D-51467 Bergisch Gladbach(DE)**

(54) **Substituierte Thienylsulfonylharnstoffe als Herbizide.**

(57) Die Erfindung betrifft neue substituierte Thienylsulfonylharnstoffe der Formel (I)

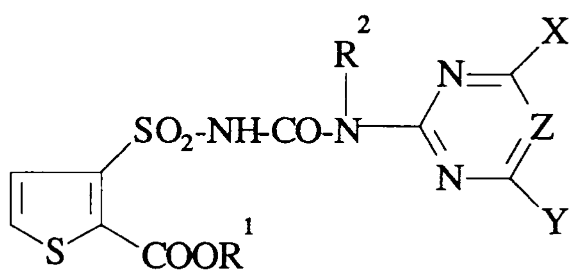

(I)

in welcher

$R^1$ für Ethyl oder n-Propyl steht,

$R^2$ für Wasserstoff oder Methyl steht,

X und Y gleich oder verschieden sind und jeweils für Wasserstoff, Hydroxy, Amino, Cyano, Halogen, $C_1$-$C_4$-Alkyl, $C_3$-$C_6$-Cycloalkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkoxy-$C_1$-$C_4$-alkoxy, $C_1$-$C_4$-Alkylthio, $C_1$-$C_4$-Alkylamino oder Di-($C_1$-$C_4$-alkyl)-amino stehen und

Z für Stickstoff, eine CH-Gruppierung oder eine C-Halogen-Gruppierung steht,

sowie Salze von Verbindungen der Formel (I), Verfahren und neue Zwischenprodukte zur Herstellung der neuen Verbindungen und deren Verwendung als Herbizide.

EP 0 590 416 A1

Die Erfindung betrifft neue substituierte Thienylsulfonylharnstoffe, Verfahren und neue Zwischenprodukte zu ihrer Herstellung sowie ihre Verwendung als Herbizide.

Es ist bereits bekannt daß bestimmte substituierte Thienylsulfonylharnstoffe, wie z.B. N-(4-Methoxy-6-methyl-1,3,5-triazin-2-yl)-N'-(2-methoxycarbonyl-thien-3-yl-sulfonyl)-harnstoff (Thifensulfuron-methyl), starke herbizide Wirksamkeit zeigen (vgl. EP-A 30142). Die Wirkung dieser Verbindungen - insbesondere ihre Verträglichkeit gegenüber Kulturpflanzen - ist jedoch nicht immer ganz zufriedenstellend.

Es wurden nun die neuen substituierten Thienylsulfonylharnstoffe der allgemeinen Formel (I)

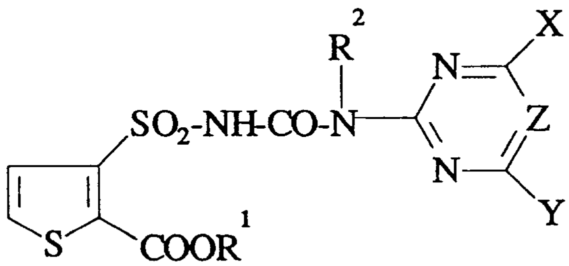

(I)

in welcher

- $R^1$ für Ethyl oder n-Propyl steht,
- $R^2$ für Wasserstoff oder Methyl steht,
- X und Y gleich oder verschieden sind und jeweils für Wasserstoff, Hydroxy, Amino, Cyano, Halogen, $C_1$-$C_4$-Alkyl, $C_3$-$C_6$-Cycloalkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkoxy-$C_1$-$C_4$-alkoxy, $C_1$-C4-Alkylthio, $C_1$-$C_4$-Alkylamino oder Di-($C_1$-$C_4$-alkyl)-amino stehen und
- Z für Stickstoff, eine CH-Gruppierung oder eine C-Halogen-Gruppierung steht,

sowie Salze von Verbindungen der Formel (I) gefunden.

Man erhält die neuen substituierten Thienylsulfonylharnstoffe der allgemeinen Formel (I), wenn man

(a) Thiophensulfonamide der allgemeinen Formel (II)

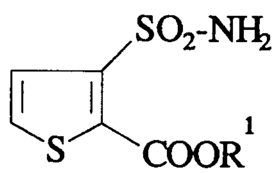

(II)

in welcher

$R^1$ die oben angegebene Bedeutung hat,

mit Chlorameisensäureestern der allgemeinen Formel (III)

$$Cl—CO—O—R^3 \quad (III)$$

in welcher

$R^3$ für $C_1$-$C_4$-Alkyl, Phenyl oder Benzyl steht,

gegebenenfalls in Gegenwart eines Säureakzeptors und gegebenenfalls in Gegenwart eines Verdünnungsmittels umsetzt und die hierbei gebildeten Urethane (Carbamate) der allgemeinen Formel (IV)

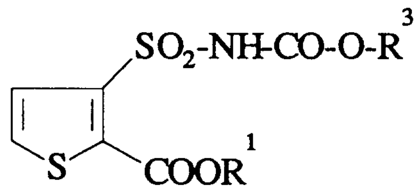

(IV)

in welcher
$R^1$ und $R^3$ die oben angegebene Bedeutung haben,
- gegebenenfalls ohne Zwischenisolierung -
mit Azinen der allgemeinen Formel (V)

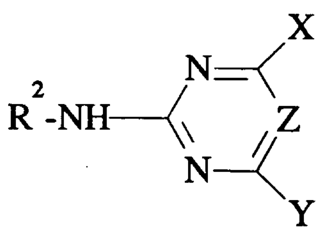

(V)

in welcher
$R^2$, X, Y und Z die oben angegebene Bedeutung haben,
gegebenenfalls in Gegenwart eines Reaktionshilfsmittels umsetzt, oder wenn man
(b) Thienylsulfonylisocyanate der allgemeinen Formel (VI)

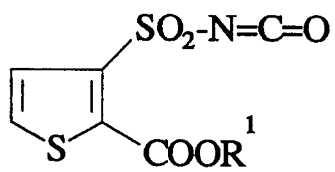

(VI)

in welcher
$R^1$ die oben angegebene Bedeutung hat,
mit Azinen der allgemeinen Formel (V)

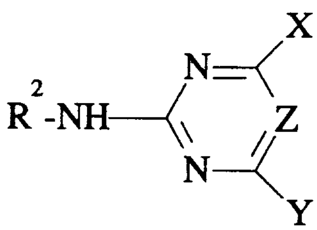

(V)

in welcher
$R^2$, X, Y und Z die oben angegebene Bedeutung haben,
gegebenenfalls in Gegenwart eines Verdünnungsmittels umsetzt.

Die neuen substituierten Thienylsulfonylharnstoffe der allgemeinen Formel (I) zeichnen sich durch starke und selektive Wirksamkeit aus.

Überraschenderweise zeigen die neuen Verbindungen der Formel (I) bei sehr guter Verträglichkeit gegenüber Kulturpflanzen wie z.B. Weizen, erheblich stärkere Wirkung gegen einige Problemunkräuter als die aus dem Stand der Technik bekannten Thienylsulfonylharnstoffe.

Gegenstand der Erfindung sind vorzugsweise Verbindungen der Formel (I), in welcher

$R^1$ für Ethyl oder n-Propyl steht,

$R^2$ für Wasserstoff oder Methyl steht,

X für Wasserstoff, Hydroxy, Amino, Cyano, Fluor, Chlor, Brom, Methyl, Ethyl, n- oder i-Propyl, n-, i-, s- oder t-Butyl, Cyclopropyl, Trifluormethyl, Methoxymethyl, Ethoxmethyl, Methoxyethyl, Ethoxyethyl, Methoxy, Ethoxy, n- oder i-Propoxy, Difluormethoxy, Trifluormethoxy, Chlorethoxy, Fluorethoxy, Dichlorethoxy, Difluorethoxy, Trichlorethoxy, Trifluorethoxy, Chlordifluorethoxy, Methoxyethoxy, Ethoxyethoxy, Methylthio, Ethylthio, n- oder i-Propylthio, Methylamino, Ethylamino, n- oder i-Propylamino, Dimethylamino oder Diethylamino steht,

Y für Wasserstoff, Hydroxy, Amino, Cyano, Fluor, Chlor, Brom, Methyl, Ethyl, Cyclopropyl, Trifluormethyl, Methoxy, Ethoxy, Difluormethoxy, Methylthio, Ethylthio, Methylamino, Ethylamino oder Dimethylamino steht und

Z für Stickstoff oder eine CH-Gruppierung steht.

Die Erfindung betrifft weiter vorzugsweise Salze, die man aus Verbindungen der Formel (I) und Basen, wie z.B. Natrium-, Kalium- oder Calcium-hydroxid, -hydrid, -amid oder -carbonat, Natrium- oder Kalium-$C_1$-$C_4$-alkanolaten, Ammioniak, $C_1$-$C_4$-Alkylaminen, Di-($C_1$-$C_4$-alkyl)-aminen oder Tri-($C_1$-$C_4$-alkyl)-aminen, erhält.

Die Erfindung betrifft insbesondere Verbindungen der Formel (I), in welcher

$R^1$ für Ethyl oder n-Propyl steht,

$R^2$ für Wasserstoff oder Methyl steht,

X für Methyl, Methoxy oder Methylthio steht,

Y für Wasserstoff, Methyl, Methoxy oder Methylthio steht und

Z für Stickstoff oder eine CH-Gruppierung steht.

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen beliebig kombiniert werden.

Verwendet man beispielsweise für die Verfahrensvariante (a) 2-Ethoxycarbonyl-thiophen-3-sulfonamid und Chlorameisensäuremethylester sowie 2-Amino-4,6-dimethoxypyrimidin als Ausgangsstoffe, so kann der Reaktionsablauf durch das folgende Formelschema skizziert werden:

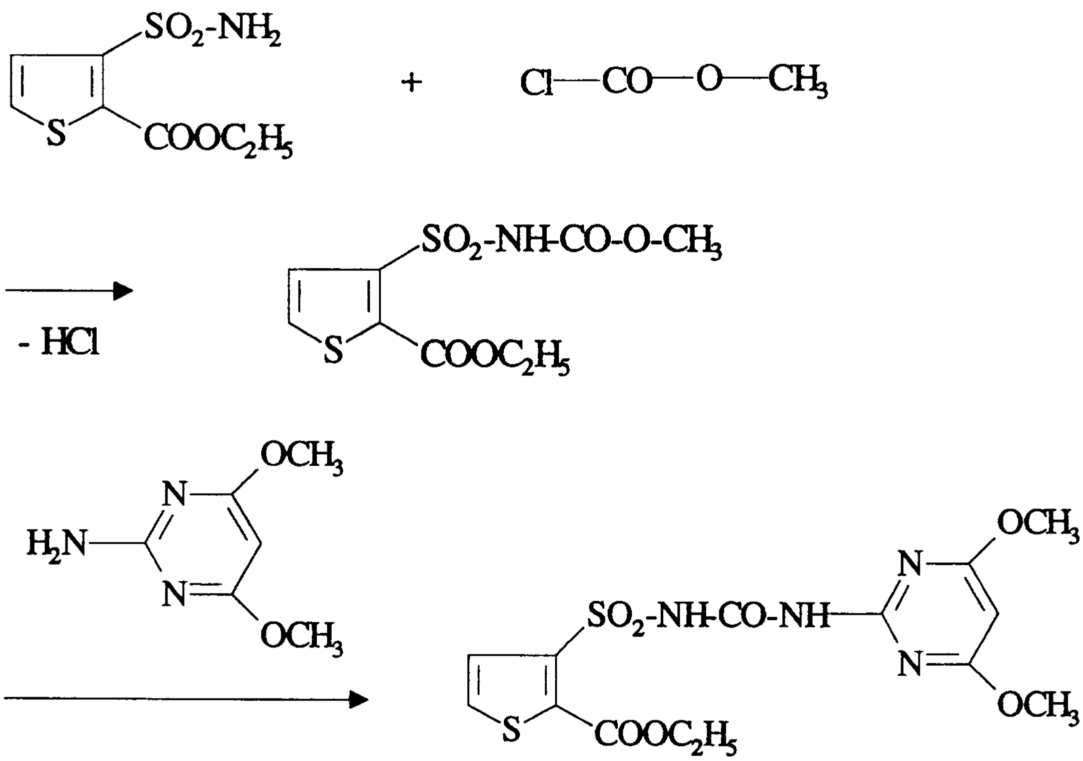


Verwendet man beispielsweise für die Verfahrensvariante (b) 2-Propoxycarbonyl-thiophen-3-yl-sulfonylisocyanat und 2-Methylamino-4,6-dimethoxy-s-triazin als Ausgangsstoffe, so kann der Reaktionsablauf durch das folgende Formelschema skizziert werden:

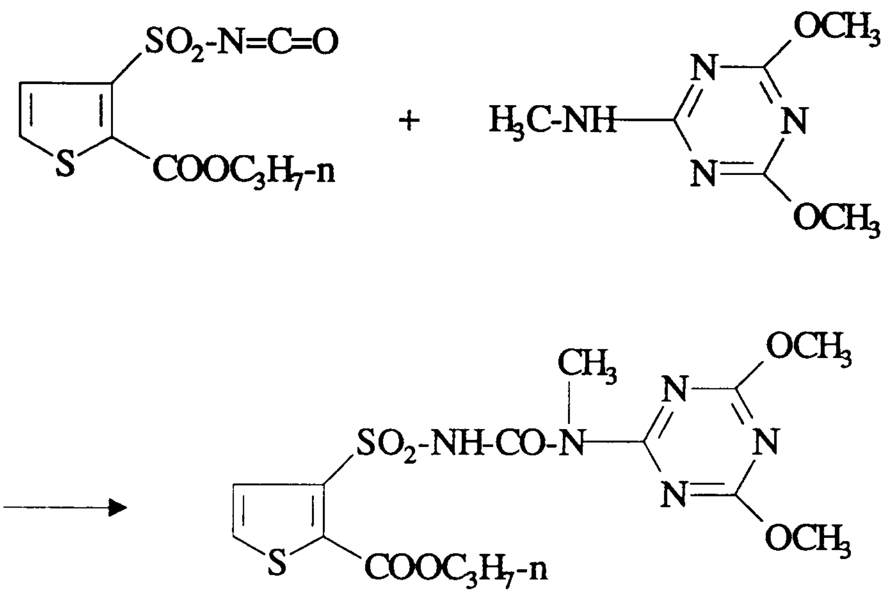

Die beim erfindungsgemäßen Herstellungsverfahren (a) als Ausgangsstoffe zu verwendenden Thiophensulfonamide - 2-Ethoxycarbonyl-thiophen-3-sulfonamid und 2-Propoxycarbonyl-thiophen-3-sulfonamid - sind noch nicht aus der Literatur bekannt und sind als neue Stoffe Gegenstand der vorliegenden Patentanmeldung.

Man erhält die neuen Thiophensulfonamide der Formel (II), wenn man entsprechende Thiophensulfonsäurechloride der allgemeinen Formel (VII)

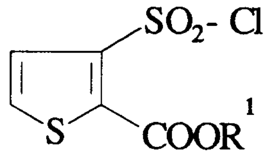

(VII)

in welcher

$R^1$ die oben angegebene Bedeutung hat,

mit Ammoniak, gegebenenfalls in Gegenwart eines Verdünnungsmittels, wie z.B. Methylenchlorid, bei Temperaturen zwischen -30 °C und +50 °C umsetzt (vgl. die Herstellungsbeispiele).

Von den Thiophensulfonsäurechloriden der Formel (VII) ist 2-Ethoxycarbonyl-thiophen-3-sulfonsäurechlorid bereits bekannt (vgl. ES-P 556919 - zitiert in Chem. Abstracts 109:93034w).

2-Propoxycarbonyl-thiophen-3-sulfonsäurechlorid ist dagegen noch nicht aus der Literatur bekannt und ist als neue Substanz Gegenstand der vorliegenden Patentanmeldung.

Man erhält das neue 2-Propoxycarbonyl-thiophen-3-sulfonsäurechlorid, wenn man 3-Amino-thiophen-2-carbonsäure-propylester mit Natriumnitrit in Gegenwart von Salzsäure bei Temperaturen zwischen -10 °C und +10 °C und dann mit Schwefeldioxid in Gegenwart eines Kupfer-Katalysators, wie z.B. Kupfer(I)-chlorid, und in Gegenwart eines Verdünnungsmittels, wie z.B. Essigsäure, bei Temperaturen zwischen 10 °C und 50 °C umsetzt (vgl. die Herstellungsbeispiele).

Der als Vorprodukt benötigte 3-Amino-thiophen-2-carbonsäure-propylester ist noch nicht aus der Literatur bekannt und ist als neue Substanz ebenfalls Gegenstand der vorliegenden Patentanmeldung.

Man erhält den neuen 3-Amino-thiophen-2-carbonsäure-propylester, wenn man Thioglycolsäure-propylester mit 2-Chlor-acrylnitril in Gegenwart eines Säureakzeptors, wie z.B. Natriumpropanolat, und in Gegenwart eines Verdünnungsmittels, wie z.B. Propanol, bei Temperaturen zwischen 0 °C und 100 °C umsetzt (vgl. die Herstellungsbeispiele).

Die beim erfindungsgemäßen Verfahren (a) zur Herstellung von Verbindungen der Formel (I) weiter als Ausgangsstoffe zu verwendenden Chlorameisensäureester sind durch die Formel (III) allgemein definiert.

In der Formel (III) steht $R^3$ vorzugsweise für Methyl, Ethyl, Phenyl oder Benzyl, insbesondere für Phenyl.

Die Ausgangsstoffe der Formel (III) sind bekannte Synthesechemikalien.

Die beim erfindungsgemäßen Verfahren (a) intermediär erhaltenen Urethane (Carbamate) der Formel (IV) sind noch nicht aus der Literatur bekannt und sind als neue Stoffe ebenfalls Gegenstand der vorliegenden Patentanmeldung.

In Formel (IV) haben $R^1$ und $R^3$ vorzugsweise bzw. insbesondere diejenigen Bedeutungen, die bereits oben im Zusammenhang mit der Beschreibung der Verbindungen der Formel (I) bzw. (III) vorzugsweise bzw. als insbesondere bevorzugt für $R^1$ und $R^3$ angegeben wurden.

Die beim erfindungsgemäßen Verfahren (a) zur Herstellung von Verbindungen der Formel (I) weiter als Ausgangsstoffe zu verwendenden Azine sind durch die Formel (V) allgemein definiert.

In Formel (V) haben $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, X, Y und Z angegeben wurden.

Die Ausgangsstoffe der Formel (V) sind bekannt und/oder können nach an sich bekannten Verfahren hergestellt werden (vgl. US-P 4299960; EP-A 121082; EP-A 125205; EP-A 126711; EP-A 152378; EP-A 158594).

Das erfindungsgemäße Verfahren (a) zur Herstellung der neuen Verbindungen der Formel (I) wird vorzugsweise unter Verwendung von Verdünnungsmitteln durchgeführt Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium-und Kalium-acetat, Alkalimetallalkoholate, wie Natrium- und Kalium-methylat, -ethylat, -propylat, isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die zweite Umsetzungsphase des erfindungsgemäßen Verfahrens, d.h. die Umsetzung mit den Azinen der Formel (V), wird vorzugsweise in Gegenwart eines Reaktionshilfsmittels durchgeführt. Als Reaktionshilfsmittel werden hierbei vorzugsweise Säuren eingesetzt, insbesondere Protonensäuren, wie Schwefelsäure, Methansulfonsäure, Benzolsulfonsäure und p-Toluolsulfonsäure.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine oder mehrere der jeweils eingesetzten Komponenten in einem größeren Überschuß zu verwenden.

Vorzugsweise werden zunächst die Thiophensulfonamide der Formel (II) mit Chlorameisensäureestern der Formel (III) umgesetzt und dann die hierbei gebildeten Urethane der Formel (IV) ohne Zwischenisolierung mit den Azinen der Formel (V) zur Umsetzung gebracht. Das Reaktionsgemisch wird dann im allgemeinen bei erhöhter Temperatur bis zum Ende der Umsetzung gerührt und anschließend auf übliche Weise aufgearbeitet (vgl. die Herstellungsbeispiele).

Die beim erfindungsgemäßen Herstellungsverfahren (b) als Ausgangsstoffe zu verwendenden Thienylsulfonylisocyanate - 2-Ethoxycarbonyl-thien-3-ylsulfonylisocyanat und 2-Propoxycarbonyl-thien-3-ylsulfonylisocyanat - sind noch nicht aus der Literatur bekannt und sind als neue Stoffe Gegenstand der vorliegenden Patentanmeldung.

Man erhält die neuen Thienylsulfonylisocyanate der Formel (VI), wenn man entsprechende Thiophensulfonamide der allgemeinen Formel (II)

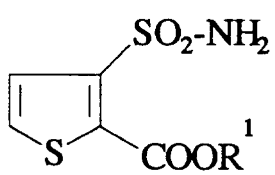

(II)

in welcher

$R^1$ die oben angegebene Bedeutung hat,

mit Phosgen, vorzugsweise in Gegenwart eines Alkylisocyanats, wie z.B. Butylisocyanat, in Gegenwart eines Reaktionshilfsmittels, wie z.B. Diazabicyclo[2.2.2]octan, und in Gegenwart eines Verdünnungsmittels, wie z.B. Xylol, bei Temperaturen zwischen 100 °C und 150 °C umsetzt und nach Ende der Umsetzung die flüchtigen Komponenten unter vermindertem Druck abdestilliert.

Das erfindungsgemäße Verfahren (b) wird weiter unter Verwendung von Azinen der allgemeinen Formel (V) durchgeführt. Es gelten hierfür die gleichen Angaben, die oben bei der Beschreibung der Ausgangsstoffe für das erfindungsgemäße Verfahren (a) hinsichtlich der Azine der Formel (V) gemacht wurden.

Das erfindungsgemäße Verfahren (b) wird vorzugsweise unter Verwendung eines Verdünnungsmittels durchgeführt. Es kommen hierbei vorzugsweise diejenigen organischen Lösungsmittel in Betracht, die oben für das erfindungsgemäße Verfahren (a) angegeben wurden.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Verdünnungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure,

Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele:

Beispiel 1

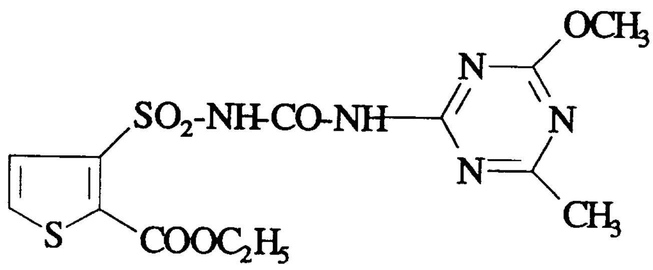

(Verfahren (a))

2,34 g (15 mMol) Chlorameisensäure-phenylester werden bei 10 °C bis 20 °C unter Rühren zu einer Mischung aus 3,53 g (15 mMol) 2-Ethoxycarbonyl-thiophen-3-sulfonamid, 3,03 g (30 mMol) Triethylamin und 60 ml Acetonitril gegeben. Nach dreißigminütigem Rühren werden zur Mischung nacheinander 1,44 g (15 mMol) Methansulfonsäure und 2,10 g (15 mMol) 2-Amino-4-methoxy-6-methyl-s-triazin gegeben. Das Reaktionsgemisch wird 30 Minuten unter Rückflüß erhitzt. Anschließend wird im Wasserstrahlvakuum eingeengt und der Rückstand mit Wasser/Methylenchlorid durchgeschüttelt. Die organische Phase wird abgetrennt, mit Natriumsulfat getrocknet und filtriert. Das Filtrat wird eingeengt, der Rückstand mit Diethylether verrührt und das kristalline Produkt durch Absaugen isoliert.

Man erhält 3,4g (57% der Theorie) N-(4-Methoxy-6-methyl-s-triazin-2-yl)-N'-(2-ethoxycarbonyl-thien-3-yl-sulfonyl)-harnstoff vom Schmelzpunkt 166 °C.

Beispiel 2

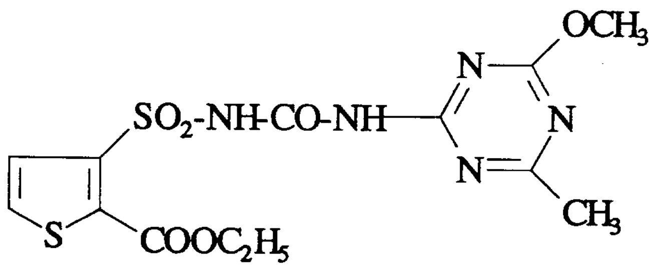

(Verfahren (b))

Eine Mischung aus 7,7 g (55 mMol) 2-Amino-4-methoxy-6-methyl-s-triazin, 14,4 g (55 mMol) 2-Ethoxycarbonyl-thien-3-yl-sulfonylisocyanat und 100 ml Acetonitril wird 4 Stunden unter Rückfluß erhitzt und dann langsam auf 20 °C abgekühlt. Das hierbei kristallin angefallene Produkt wird durch Absaugen isoliert.

Man erhält 15,6 g (71% der Theorie) N-(4-Methoxy-6-methyl-s-triazin-2-yl)-N'-(2-ethoxycarbonyl-thien-3-yl-sulfonyl)-harnstoff vom Schmelzpunkt 166 °C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

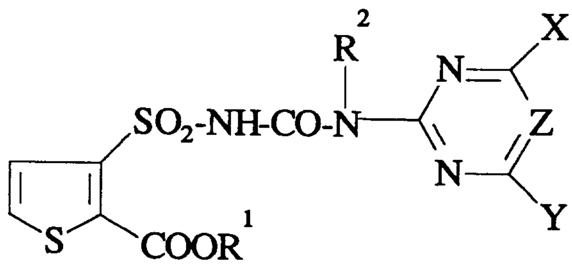

(I)

**Tabelle 1:** Beispiele für die Verbindungen der Formel (I)

| Bsp.-Nr. | $R^1$ | $R^2$ | X | Y | Z | Schmelzpunkt (°C) |
|---|---|---|---|---|---|---|
| 3 | $C_2H_5$ | H | $OCH_3$ | H | N | 203 |
| 4 | $C_2H_5$ | H | $OCH_3$ | $OCH_3$ | N | 180 |
| 5 | $C_2H_5$ | H | $OCH_3$ | $OCH_3$ | CH | 153 |
| 6 | $C_2H_5$ | H | $OCH_3$ | 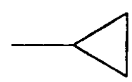 | N | 173 |
| 7 | $C_2H_5$ | H | $SCH_3$ | 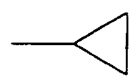 | N | 143 |
| 8 | $C_2H_5$ | H | i-$C_3H_7$ | $SCH_3$ | N | 143 |
| 9 | n-$C_3H_7$ | H | $OCH_3$ | $CH_3$ | N | 155 |
| 10 | n-$C_3H_7$ | H | $OCH_3$ | $CH_3$ | CH | 153 |
| 11 | n-$C_3H_7$ | H | $OCH_3$ | H | N | 187 |
| 12 | n-$C_3H_7$ | H | $OCH_3$ | $OCH_3$ | N | 162 |
| 13 | $C_2H_5$ | H | $CH_3$ | $CH_3$ | N | 196 |
| 14 | $C_2H_5$ | H | $SCH_3$ | $CH_3$ | N | 184 |
| 15 | n-$C_3H_7$ | H | $CH_3$ | $CH_3$ | N | 169 |
| 16 | n-$C_3H_7$ | H | $SCH_3$ | $CH_3$ | N | 173 |
| 17 | n-$C_3H_7$ | H | $CH_3$ | $CH_3$ | CH | 159 |
| 18 | n-$C_3H_7$ | H | $CH_3$ | H | CH | 186 |

**Tabelle 1** - Fortsetzung

| Bsp.-Nr. | $R^1$ | $R^2$ | X | Y | Z | Schmelzpunkt (°C) |
|---|---|---|---|---|---|---|
| 19 | $n\text{-}C_3H_7$ | H | $OCH_3$ | $OCH_3$ | CH | 132 |
| 20 | $C_2H_5$ | H | $OCH_3$ | $CH_3$ | CH | 182 |
| 21 | $C_2H_5$ | H | $CH_3$ | $CH_3$ | CH | 166 |
| 22 | $C_2H_5$ | H | $CH_3$ | H | CH | 188 |
| 23 | $C_2H_5$ | $CH_3$ | $OCH_3$ | $CH_3$ | N | 137 |
| 24 | $C_2H_5$ | $CH_3$ | $OCH_3$ | $OCH_3$ | N | 138 |
| 25 | $C_2H_5$ | H | $OCH_3$ | $CH_3$ | N | 200 (Na-Salz) |
| 26 | $C_2H_5$ | H | $t\text{-}C_4H_9$ | H | CH | 185 |
| 27 | $C_2H_5$ | H | $OCH_3$ | $CH_3$ | N | 149 |
| 28 | $C_2H_5$ | H | $OCH_3$ | $C_2H_5$ | N | 173 |
| 29 | $C_2H_5$ | H | $CH_3$ | $C_2H_5$ | CH | 207 |
| 30 | $C_2H_5$ | H | $i\text{-}C_4H_9$ | H | CH | 176 |
| 31 | $C_2H_5$ | H | $t\text{-}C_4H_9$ | $SCH_3$ | N | 195 |
| 32 | $C_2H_5$ | H | $SCH_3$ | $N(CH_3)_2$ | N | 165 |
| 33 | $C_2H_5$ | H | $t\text{-}C_4H_9$ | OH | N | 250 |
| 34 | $C_2H_5$ | $CH_3$ | CN | $OCH_3$ | N | 139 |

**Tabelle 1** - Fortsetzung

| Bsp.-Nr. | $R^1$ | $R^2$ | X | Y | Z | Schmelzpunkt (°C) |
|---|---|---|---|---|---|---|
| 35 | n-$C_3H_7$ | $CH_3$ | $OCH_3$ | $CH_3$ | N | |
| 36 | n-$C_3H_7$ | $CH_3$ | $OCH_3$ | $OCH_3$ | N | |
| 37 | $C_2H_5$ | H | $CH_3$ | $N(CH_3)_2$ | N | 143 |
| 38 | $C_2H_5$ | H | t-$C_4H_9$ | $OCH_3$ | N | |
| 39 | $C_2H_5$ | H | $OCH_3$ | $N(CH_3)_2$ | N | |
| 40 | $C_2H_5$ | H | CN | $OCH_3$ | N | |
| 41 | $C_2H_5$ | H | Cl | Cl | CH | 176 |
| 42 | $C_2H_5$ | H | $OCH_3$ | Cl | CH | |
| 43 | $C_2H_5$ | H | Cl | $OCH_3$ | CH | |
| 44 | $C_2H_5$ | H | $OCH_3$ | H | CH | |
| 45 | $C_2H_5$ | H | H | H | CCl | |

Die oben als neue Verbindungen genannten Zwischenprodukte können durch die folgenden Formeln wiedergegeben werden:

Die Formel (IX),

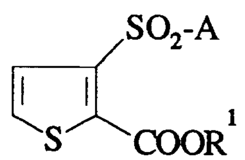

(IX)

in welcher

A für $NH_2$, NH-CO-O-$R^3$ oder N=C=O steht,

$R^1$ für Ethyl oder n-Propyl steht und

$R^3$ für $C_1$-$C_4$-Alkyl, Phenyl oder Benzyl steht,

repräsentiert die Thiophenderivate der obigen Formeln (II), (IV) und (VI).

Die Formel (X),

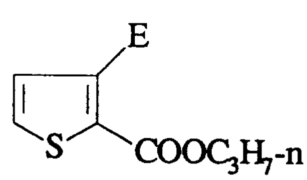

(X)

in welcher
E für $NH_2$ oder $SO_2Cl$ steht,
repräsentiert die Thiophenderivate der Formeln (VIII-1) und (VII-1).

Ausgangsstoffe der Formel (II):

Beispiel (II-1)

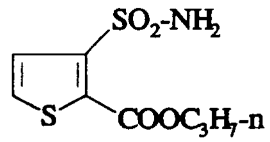

(II-1)

46 g Ammoniak werden unter Rühren in eine auf 0 °C bis 10 °C abgekühlte Lösung von 50 g (187 mMol) 2-Propoxycarbonyl-thiophen-3-sulfonsäurechlorid in 500 ml Methylenchlorid eingeleitet. Das Reaktionsgemisch wird 15 Stunden bei 20 °C gerührt und dann filtriert. Das Filtrat wird eingeengt, der Rückstand mit Diethylether verrührt und das kristallin angefallene Produkt durch Absaugen isoliert.

Man erhält 31 g (67% der Theorie) 2-Propoxycarbonyl-thiophen-3-sulfonamid vom Schmelzpunkt 79 °C.

Beispiel (II-2)

Analog erhält man auch 2-Ethoxycarbonyl-thiophen-3-sulfonamid vom Schmelzpunkt 136 °C.

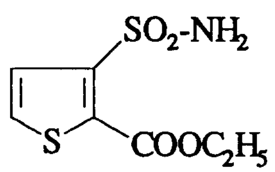

(II-2)

Ausgangsstoffe der Formel (VII):

Beispiel (VII-1)

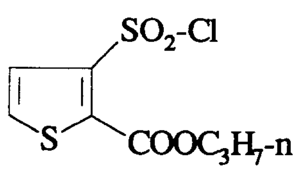

(VII-1)

Eine Lösung von 76 g (1,10 Mol) Natriumnitrit in 140 ml Wasser wird bei -5 °C bis 0 °C innerhalb von 60 Minuten zu er Lösung von 185 g (1,00 Mol) 3-Amino-thiophen-2-carbonsäure-propylester in 630 ml Salzsäure tropfenweise gegeben. Das Gemisch wird noch 60 Minuten bei 0 °C gerührt und dann tropfenweise bei 15 °C zu einer Lösung von 600 g Schwefeldioxid in 1200 ml Essigsäure, zu welcher man vorher 115 ml einer gesättigten wäßrigen Lösung von Kupfer(I)-chlorid gegeben hat, gegeben. Das Reaktionsgemisch wird dann 15 Stunden bei 20 °C gerührt, dann mit Eiswasser auf etwa das doppelte Volumen verdünnt und zweimal mit Methylenchlorid extrahiert. Die vereinigten organischen Extraktionslösungen werden mit Natriumsulfat getrocknet und filtriert. Vom Filtrat wird das Lösungsmittel im Wasserstrahlvakuum sorgfältig abdestilliert.

Man erhält 205 g (76% der Theorie) 2-Propoxycarbonyl-thiophen-3-sulfonsäurechlorid als öligen Rückstand, welcher ohne weitere Reinigung zur nächsten Reaktionsstufe eingesetz werden kann.

Beispiel (VII-2)

Analog erhält man auch 2-Ethoxycarbonyl-thiophen-3-sulfonsäurechlorid

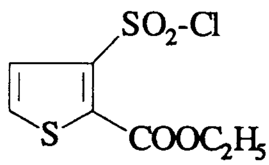

(VII-2)

Herstellung von 3-Amino-thiophen-2-carbonsäure-propylester:

Beispiel (VIII-1)

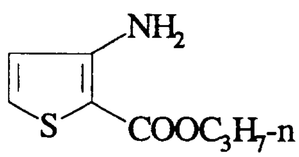

(VIII-1)

Durch Umsetzung von 2,6 g (113 mMol) Natrium mit Propanol (100 ml) bei 50 °C wird eine Natriumpropylatlösung hergestellt. Zu dieser Lösung wird bei 20 °C eine Lösung von 6,7 g (50 mMol) Thioglycolsäure-n-propylester in 20 ml Propanol getropft und dann wird bei 25 °C bis 30 °C (Außenkühlung erforderlich) eine Lösung von 4,4 g (50 mMol) 2-Chlor-acrylnitril in 20 ml Propanol eindosiert. Das Reaktionsgemisch wird 20 Stunden bei 20 °C gerührt, dann auf etwa 1/3 des Volumens eingeengt, dann mit 100 ml Wasser verdünnt und dreimal mit je 100 ml Diethylether extrahiert. Die vereinigten organischen Extraktionslösungen werden mit Wasser gewaschen, mit Natriumsulfat getrocknet und filtriert. Vom Filtrat wird das Lösungsmittel im Wasserstrahlvakuum sorgfältig abdestilliert.

Man erhält 5 g (54% der Theorie) 3-Amino-thiophen-2-carbonsäure-propylester als öligen Rückstand, welcher direkt weiter umgesetzt werden kann.

Anwendungsbeispiele:

In den folgenden Anwendungsbeispielen wird die nachstehend aufgeführte Verbindung als Vergleichssubstanz herangezogen:

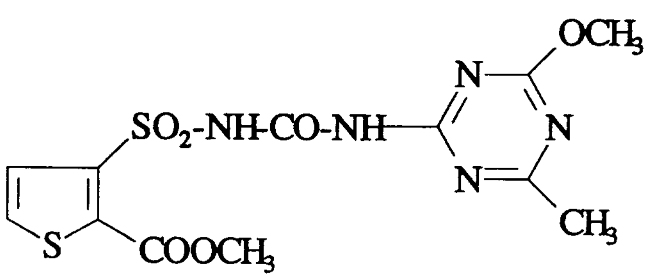

(A)

N-(4-Methoxy-6-methyl-1,3,5-triazin-2-yl)-N'-(2-methoxycarbonyl-thien-3-yl-sulfonyl)-harnstoff (Thifensulfuron-methyl)
(bekannt aus EP-A 30142).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100% = totale Vernichtung

In diesem Test zeigen beispielsweise die Verbindungen gemäß Herstellungsbeispiel 1, 4, 9, 10, 12, 14, 16, 17, 19, 20, 21, 22 und 23 bei teilweise sehr guter Verträglichkeit gegenüber Kulturpflanzen, wie z.B. Weizen, sehr starke Wirkung gegen Unkräuter. Dies gilt insbesondere auch für die Verbindung gemäß Herstellungsbeispiel 28.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
O % = keine Wirkung (wie unbehandelte Kontrolle)
100 % = totale Vernichtung

In diesem Test zeigen beispielsweise die Verbindungen gemäß Herstellungsbeispiel 1, 3, 4, 5, 9, 10, 12, 13, 14, 17, 19, 20, 21, 22 und 23 bei durchweg sehr guter Verträglichkeit gegenüber Kulturpflanzen, wie z.B. Weizen, sehr starke Wirkung gegen Unkräuter. Entsprechendes gilt auch für die Verbindung gemäß Herstellungsbeispiel 28.

**Patentansprüche**

**1.** Substituierte Thienylsulfonylharnstoffe der allgemeinen Formel (I)

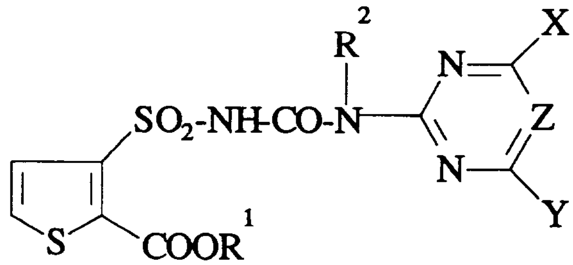

(I)

in welcher

$R^1$ für Ethyl oder n-Propyl steht,

$R^2$ für Wasserstoff oder Methyl steht,

X und Y gleich oder verschieden sind und jeweils für Wasserstoff, Hydroxy, Amino, Cyano, Halogen, $C_1$-$C_4$-Alkyl, $C_3$-$C_6$-Cycloalkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkoxy-$C_1$-$C_4$-alkoxy, $C_1$-$C_4$-Alkylthio, $C_1$-$C_4$-Alkylamino oder Di-($C_1$-$C_4$-alkyl)-amino stehen und

Z für Stickstoff, eine CH-Gruppierung oder eine C-Halogen-Gruppierung steht,

sowie Salze von Verbindungen der Formel (I).

**2.** Verbindungen der Formel (I) gemäß Anspruch 1, dadurch gekennzeichnet, daß darin

$R^1$ für Ethyl oder n-Propyl steht,

$R^2$ für Wasserstoff oder Methyl steht,

X für Wasserstoff, Hydroxy, Amino, Cyano, Fluor, Chlor, Brom, Methyl, Ethyl, n- oder i-Propyl, n-, i-, s- oder t-Butyl, Cyclopropyl, Trifluormethyl, Methoxymethyl, Ethoxymethyl, Methoxyethyl, Ethoxyethyl, Methoxy, Ethoxy, n- oder i-Propoxy, Difluormethoxy, Trifluormethoxy, Chlorethoxy, Fluorethoxy, Dichlorethoxy, Difluorethoxy, Trichlorethoxy, Trifluorethoxy, Chlordifluorethoxy, Methoxyethoxy, Ethoxyethoxy, Methylthio, Ethylthio, n- oder i-Propylthio, Methylamino, Ethylamino, n- oder i-Propylamino, Dimethylamino oder Diethylamino steht,

Y für Wasserstoff, Hydroxy, Amino, Cyano, Fluor, Chlor, Brom, Methyl, Ethyl, Cyclopropyl, Trifluormethyl, Methoxy, Ethoxy, Difluormethoxy, Methylthio, Ethylthio, Methylamino, Ethylamino oder Dimethylamino steht und

Z für Stickstoff oder eine CH-Gruppierung steht.

**3.** Verbindungen der Formel (I) gemäß Anspruch 1, dadurch gekennzeichnet, daß darin

$R^1$ für Ethyl oder n-Propyl steht,

$R^2$ für Wasserstoff oder Methyl steht,

X für Methyl, Methoxy oder Methylthio steht,

Y für Wasserstoff, Methyl, Methoxy oder Methylthio steht und

Z für Stickstoff oder eine CH-Gruppierung steht.

**4.** Verfahren zur Herstellung von substituierten Thienylsulfonylharnstoffen der Formel (I) gemäß Anspruch 1, dadurch gekennzeichnet, daß man

(a) Thiophensulfonamide der allgemeinen Formel (II)

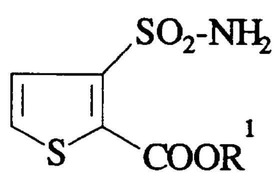

(II)

in welcher

$R^1$ die in Anspruch 1 angegebene Bedeutung hat,

mit Chlorameisensäureestern der allgemeinen Formel (III)

Cl—CO—O—$R^3$ (III)

in welcher

$R^3$ für $C_1$-$C_4$-Alkyl, Phenyl oder Benzyl steht,

gegebenenfalls in Gegenwart eines Säureakzeptors und gegebenenfalls in Gegenwart eines Verdünnungsmittels umsetzt und die hierbei gebildeten Urethane (Carbamate) der allgemeinen Formel (IV)

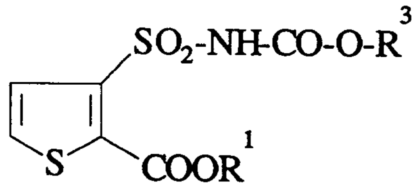

(IV)

in welcher

$R^1$ und $R^3$ die in Anspruch 1 angegebene Bedeutung haben,

- gegebenenfalls ohne Zwischenisolierung -

mit Azinen der allgemeinen Formel (V)

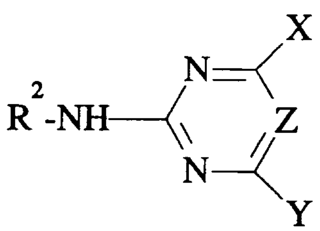

(V)

in welcher

$R^2$, X, Y und Z die in Anspruch 1 angegebene Bedeutung haben,

gegebenenfalls in Gegenwart eines Reaktionshilfsmittels umsetzt, oder daß man

(b) Thienylsulfonylisocyanate der allgemeinen Formel (VI)

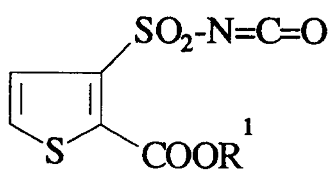

(VI)

in welcher

$R^1$ die oben angegebene Bedeutung hat,

mit Azinen der allgemeinen Formel (V)

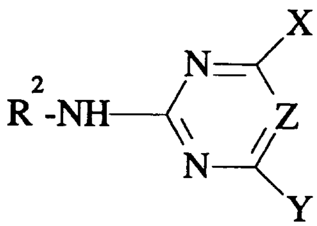

(V)

in welcher

$R^2$, X, Y und Z die oben angegebene Bedeutung haben,

gegebenenfalls in Gegenwart eines Verdünnungsmittels umsetzt.

**5.** Herbizide Mittel, gekennzeichnet durch einen Gehalt an mindestens einer Verbindung der Formel (I) gemäß Anspruch 1.

**6.** Verwendung von Verbindungen der allgemeinen Formel (I) gemäß Anspruch 1 zur Bekämpfung von unerwünschtem Pflanzenwachstum.

**7.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**8.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

**9.** Thiophenderivate der Formel (IX),

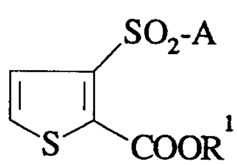

(IX)

in welcher

A für $NH_2$, NH-CO-O-$R^3$ oder N = C = O steht,

$R^1$ für Ethyl oder n-Propyl steht und

$R^3$ für $C_1$-$C_4$-Alkyl, Phenyl oder Benzyl steht.

**10.** Thiophenderivate der Formel (X),

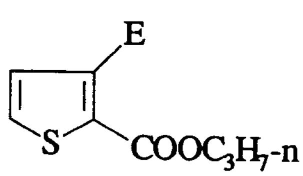

(X)

in welcher

E für $NH_2$ oder $SO_2Cl$ steht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | EP-A-0 207 609 (E.I. DU PONT DE NEMOURS AND COMPANY) 7. Januar 1987<br>* Seite 93, erste 5 Verbindungen; Seite 118, Verbindung 31 *<br>--- | 1-8 | C07D409/12<br>A01N47/36<br>C07D333/38 |
| X,D | EP-A-0 030 142 (E.I. DU PONT DE NEMOURS AND COMPANY) 10. Juni 1981<br>* Seite 65, 7te Verbindung von unten; Seite 161, erste Verbindung *<br>--- | 1-9 | |
| X | EP-A-0 164 269 (E.I. DU PONT DE NEMOURS AND COMPANY) 11. Dezember 1985<br>* Seiten 44, 46, 48 *<br>--- | 1-8 | |
| A | EP-A-0 097 122 (CIBA-GEIGY AG) 28. Dezember 1983<br>* Seite 28 *<br>--- | 1-8 | |
| P,X | CHEMICAL ABSTRACTS, vol. 119, no. 17, 1993, Columbus, Ohio, US;<br>abstract no. 180647y,<br>M. ISHIZAKI ET AL.<br>Seite 773 ;<br>* Zusammenfassung *<br>& JP-A-05 117 263 (TOKUYAMA SODA) 14. Mai 1993<br>----- | 10 | RECHERCHIERTE SACHGEBIETE (Int.Cl.5)<br>C07D |

Der vorliegende Recherchenbericht wurde für alle Patentansprüche erstellt

| Recherchenort | Abschlußdatum der Recherche | Prüfer |
|---|---|---|
| DEN HAAG | 3. Dezember 1993 | De Jong, B |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument